(19) Europäisches Patentamt
European Patent Office
Office européen des brevets

(11) Publication number: **0 679 645 A1**

(12) # EUROPEAN PATENT APPLICATION

(21) Application number: **95111314.1**

(51) Int. Cl.[6]: **C07D 239/48**, A61K 31/505

(22) Date of filing: **31.05.91**

This application was filed on 19 - 07 - 1995 as a divisional application to the application mentioned under INID code 60.

(30) Priority: **01.06.90 GB 9012316**

(43) Date of publication of application: **02.11.95 Bulletin 95/44**

(60) Publication number of the earlier application in accordance with Art.76 EPC: **0 459 819**

(84) Designated Contracting States: **AT BE CH DE DK ES FR GB GR IT LI LU NL SE**

(71) Applicant: **THE WELLCOME FOUNDATION LIMITED**
**Unicorn House**
**160 Euston Road**
**London NW1 2BP (GB)**

(72) Inventor: **Leach, Michael John**
**Langley Court**
**Beckenham,**
**Kent BR3 3BS (GB)**
Inventor: **Nobbs, Malcolm Stuart**
**Langley Court**
**Beckenham,**
**Kent BR3 3BS (GB)**
Inventor: **Iyer, Ramachandran**
**Langley Court**
**Beckenham,**
**Kent BR3 3BS (GB)**
Inventor: **Yeates, Clive Leonard**
**Langley Court**
**Beckenham,**
**Kent BR3 3BS (GB)**
Inventor: **Skone, Philip Alan**
**Langley Court**
**Beckenham,**
**Kent BR3 3BS (GB)**

(74) Representative: **Woods, Geoffrey Corlett**
**J.A. KEMP & CO.**
**14 South Square**
**Gray's Inn**
**London WC1R 5LX (GB)**

(54) **Pharmacologically active CNS pyridmidin compounds.**

(57) A series of phenylpyrimidines and acid addition salts thereof are inhibitors of extracellular glutamate release. They have negligible effect against the enzyme dihydrofolate reductase. They are useful in the treatment or prophylaxis in a mammal of a neurodegenerative or other neurological disorder of the central nervous system, the aetiology of which includes excessive release of neurotransmitter glutamate.



Rank Xerox (UK) Business Services
(3.10/3.09/3.3.4)

This present invention relates to a series of substituted 5-aryl pyrimidines, to methods for their manufacture, to pharmaceutical formulations containing them and to their use in therapy, in particular the treatment of a range of CNS disorders and disease states.

Glutamate is an excitatory amino acid which functions as a neurotransmitter. However, when its extracellular concentration is sufficiently high, glutamate acts as a powerful neurotoxin, capable of killing neurones in the central nervous system, (Rothman & Olney (1986) Prog.Brain.Res., 63, 69). The neurotoxic effect of glutamate has been implicated in a number of central nervous system disorders and disease states including cerebral ischaemic damage and chronic neurodegenerative disorders, such an Alzheimer's disease, motor system disorders, and Huntington's chorea, (Meldrum Clinical Science (1985) 68 113-122). In addition, glutamate has been implicated in other neurological disorders such as epilepsy, manic depression, depression, schizophrenia, high pressure neurological syndrome, chronic pain, trigeminal neuralgia and migraine.

European patent application No. 89312723.3 (EP-A-0372934) discloses a generic class of substituted phenyl-pyrimidines for use in the manufacture of a medicament for treating or preventing CNS disorders or diseases of a mammal.

The present invention provides a compound selected from the following group and salts of such compounds:-

1. 2,4-Diamino-5-(3,5-dichlorophenyl)-6-trifluoromethyl pyrimidine;
2. 2,4-Diamino-6-methyl-5-(2,3,6-trichlorophenyl)pyrimidine;
3. 2,4-Diamino-5-(4-amino-2,3,5-trichlorophenyl)pyrimidine;
4. 4-Amino-2-(4-methyl-1-piperazinyl)-6-methyl-5-(3,4,5-trichlorophenyl)pyrimidine;
5. 2,4-Diamino-5-(3,4,5-trichlorophenyl)-6-methyl pyrimidine;
6. 4-Amino-2-diethylamino-5-(2,3,5-trichlorophenyl)pyrimidine;
7. 4-Amino-2-(2-iso-propylamino)-5-(2,3,5-trichlorophenyl)pyrimidine;
8. 4-Amino-5-(3,5-dichlorophenyl)-2-(4-methyl-1-piperazinyl)pyrimidine;
9. 2,4-Diamino-5-(4-iodo-2,3,5-trichlorophenyl)pyrimidine;
10. 4-Amino-2-(4-methyl-4-oxide-1-piperazinyl)-5-(2,3,5-trichlorophenyl)-6-trifluoromethyl-pyrimidine;
11. 4-Amino-5-(3,5-dichlorophenyl)-6-methoxymethyl-2-(4-methyl-1-piperazinyl)pyrimidine;
12. 2,4-Diamino-5[4-(N',N'-dimethylsulphamoyl)-2,3,5-trichlorophenyl]pyrimidine;
13. 2-Amino-5-(2,3-dichlorophenyl)-4-(1-piperidinyl)-pyrimidine;
14. 4-Amino-5-(3,5-dichlorophenyl)-2-(4-methyl-1-piperazinyl)-6-trifluoromethyl pyrimidine;
15. 2,4-Diamino-5-(2,3,4,5-tetrachlorophenyl)pyrimidine;
16. cis 4-Amino-2-(3,5-dimethyl-1-piperazinyl)-5-(2,3,5-trichlorophenyl)pyrimidine;
17. 4-Amino-2-(4-n-propyl-1-piperazinyl)-5-(2,3,5-trichlorophenyl)pyrimidine;
18. 4-Amino-2-(4-methyl-4-oxide-1-piperazinyl)-5-(2,3,5-trichlorophenyl)pyrimidine;
19. 2,4-Diamino-5-(3,5-dichloro-2-nitrophenyl)-6-methyl-pyrimidine;
20. 2-Amino-5-(2,3-dichlorophenyl)-4-(4-methyl-1-piperazinyl)pyrimidine;
21. 2-Amino-5-(2,3-dichlorophenyl)-4-(N-morpholino)pyrimidine;
22. 4-Amino-2-(4-benzyl-1-piperazinyl)-5-(2,3,5-trichlorophenyl)pyrimidine;
23. 4-Amino-2-(N-morpholino)-5-(3,5-dichlorophenyl)-6-methyl pyrimidine;
24. 4-Amino-2-(N-morpholino)-5-(3,5-dichlorophenyl)-6-trifluoromethyl pyrimidine;
25. 2,4-Diamino-5-(3,5-dichloro-4-aminophenyl)pyrimidine;
26. 2,4-Diamino-5-(3,5-dichloro-4-aminophenyl)-6-methyl pyrimidine;
27. 2,4-Diamino-5-(3,5-dichloro-4-aminophenyl)-6-trifluoromethyl pyrimidine;
28. 4-Amino-2-(4-n-butyl-1-piperazinyl)-5-(2,3,5-trichlorophenyl)pyrimidine;
29. 4-Amino-2-(4-ethyl-1-piperazinyl)-5-(2,3,5-trichlorophenyl)pyrimidine;
30. 4-Amino-2-(4-isopropyl-1-piperazinyl)-5-(2,3,5-trichlorophenyl)pyrimidine;
31. 4-Amino-2-(4-cyclopropyl-1-piperazinyl)-5-(2,3,5-trichlorophenyl)pyrimidine;
32. 4-Amino-2-(4-ethyl-1-piperazinyl)-5-(3,5-dichlorophenyl)-6-trifluoromethyl pyrimidine;
33. 4-Amino-2-(4-isopropyl-1-piperazinyl)-5-(3,5-dichlorophenyl)-6-trifluoromethyl pyrimidine;
34. 4-Amino-2-(4-cyclopropyl-1-piperazinyl)-5-(3,5-dichlorophenyl)-6-trifluoromethyl pyrimidine;
35. 4-Amino-2-(4-methyl-1-piperazinyl)-5-(2,4,5-trichlorophenyl)pyrimidine.

The present invention further provides certain groups of compounds encompassing certain of the compounds listed above. Accordingly the invention provides a first group of compounds of formula (I) and salts thereof

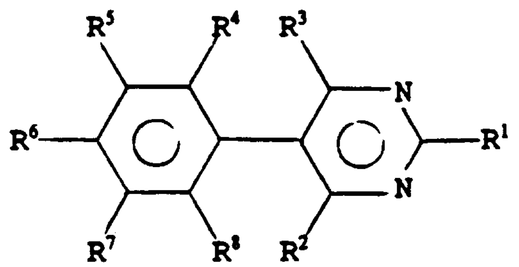

(I)

wherein $R^1$ and $R^2$, which may be the same or different, each represent $-NR^{13}R^{14}$ where $R^{13}$ and $R^{14}$ may each independently represent hydrogen or alkyl or, taken together with the nitrogen atom to which they are attached form a heterocyclic ring, optionally substituted by one or more alkyl or arylalkyl groups and optionally containing a further heteroatom, e.g. N-morpholino, N-piperazinyl, N-4-($C_{1-4}$alkyl)piperazin-1-yl;
$R^3$ is hydrogen, haloalkyl (e.g. trifluoromethyl) alkoxymethyl (e.g. methoxymethyl), $C_{1-4}$alkyl;
$R^4$ is hydrogen, nitro or halo;
$R^5$ is hydrogen or halo;
$R^6$ is hydrogen, halo, amino, alkylamino, dialkylamino;
$R^7$ is hydrogen or halo;
$R^8$ is hydrogen or halo.

According to one embodiment of the compounds of formula (I), $R^1$ represents amino, N-alkylamino, N,N-dialkylamino, N-morpholino, N-piperazinyl, N-4-alkylpiperazin-1-yl or N-4-(arylalkyl)piperazin-1-yl; $R^2$ represents amino, N-morpholino or N-piperidino.

The invention also provides a second group of compounds of formula (I) and salts thereof wherein $R^1$, $R^2$, $R^3$, $R^4$ and $R^8$ are as defined above and $R^5$, $R^6$ and $R^7$ are each chloro or iodo.

The invention also provides a third group of compounds of formula (I) and salts thereof wherein $R^1$, $R^2$, $R^3$, $R^4$ and $R^8$ are as defined above, $R^6$ is amino and $R^5$ and $R^7$ are chloro.

The invention also provides a fourth group of compounds of formula (I) and salts thereof wherein $R^1$, $R^2$, $R^3$ and $R^6$ are as defined above, $R^4$ and $R^8$ are both hydrogen, and $R^5$ and $R^7$ are both chloro, with the proviso that when $R^6$ is hydrogen, $R^2$ is amino, and $R^1$ is amino or N-methylpiperazinyl, then $R^3$ is other than methyl or methoxymethyl.

Particularly preferred compounds according to the present invention are:
2,4-diamino-5-(4-amino-2,3,5-trichlorophenyl)pyrimidine;
4-amino-5-(3,5-dichlorophenyl)-2-(4-methyl-1-piperazinyl)pyrimidine;
4-amino-2-(4-n-propyl-1-piperazinyl)-5-(2,3,5-trichlorophenyl)pyrimidine;
4-amino-2-(4-ethyl-1-piperazinyl)-5-(2,3,5-trichlorophenyl)pyrimidine;
and salts thereof. The above compounds are potent inhibitors of extracellular glutamate release, and have negligible effect against the enzyme dihydrofolate reductase. The second named compound has been shown to have a long half-life in the rat.

The present invention includes the compounds defined above, i.e. the compounds specifically named and the compounds of formula (I), in the form of salts, in particular acid addition salts. Suitable acid addition salts include those formed with both organic or inorganic acids. Such acid addition salts will normally be pharmaceutically acceptable although salts with non-pharmaceutically acceptable acids may be of utility in the preparation and purification of the compound in question. Thus, preferred salts include those formed from hydrochloric, hydrobromic, sulphuric, citric, tartaric, phosphoric, lactic, pyruvic, acetic, succinic, oxalic, fumaric, maleic, oxaloacetic, methanesulphonic, ethanesulphonic, p-toluenesulphonic, benzenesulphonic and isethionic acids. Salts of the compounds according to the invention can be made by reacting the appropriate compound in the form of the free base with the appropriate acid.

The compounds of the present invention may be used for the treatment or prophylaxis in a mammal of a neurodegenerative or other neurological disorder of the central nervous system, the aetiology of which includes excessive release of neurotransmitter glutamate.

Such disorders can be either acute or chronic. Acute conditions include cerebral ischaemic damage which may arise from a variety of causes including stroke, cardiac arrest, cardiac bypass surgery, neonatal anoxia and hypoglycaemia; and also physical injury or trauma of the spinal cord or brain. Chronic disorders include Alzheimer's disease, Huntington's chorea, Olivopontocerebrellar atrophy and motor system disorders. Other neurological conditions which may be treated with a compound according to the invention include depression, manic depression, schizophrenia, chronic pain, epilepsy, trigeminal neuralgia and

migraine.

According to a further aspect, the present invention provides a method for the treatment or prophylaxis in a mammal, including man, of a neurodegenerative or other neurological disorder of the central nervous system, the aetiology of which includes excessive release of neurotransmitter glutamate which comprises administering to the said mammal a non-toxic effective amount of a compound according to the present invention as defined above.

The invention also provides a compound according to the present invention as defined above for use in therapy, in particular the treatment or prophylaxis of a neurodegenerative or other neurological disorder of the central nervous system, the aetiology of which includes excessive release of the neurotransmitter glutamate. The invention further provides the use of a compound according to the invention as defined above for the manufacture of a medicament for the treatment or prophylaxis of a neurodegenerative or other neurological disorder of the central nervous system, the aetiology of which includes excessive release of neurotransmitter glutamate.

While it is possible for the compounds of the present invention to be administered as the raw chemical, it is preferable to present them as a pharmaceutical formulation. The formulations of the present invention comprise a compound of the present invention, as above defined, or a pharmaceutically acceptable salt thereof together with one or more pharmaceutically acceptable carriers therefor and optionally other therapeutic ingredients. The carrier(s) must be 'acceptable' in the sense of being compatible with the other ingredients of the formulation and not deleterious to the recipient thereof.

The formulations include those suitable for oral, parenteral (including subcutaneous, intradermal, intramuscular and intravenous), rectal and topical (including dermal, buccal and sublingual) administration although the most suitable route may depend upon for example the condition and disorder of the recipient. The formulations may conveniently be presented in unit dosage form and may be prepared by any of the methods well known in the art of pharmacy. All methods include the step of bringing into association a compound of the present invention as herein defined or a pharmaceutically acceptable salt thereof ("active ingredient") with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example, water-for-injection, immediately prior to use. Extemporaneous injection solutions and suspensions may be prepared from sterile powders, granules and tablets of the kind previously described.

Formulations for rectal administration may be presented as a suppository with the usual carriers such as cocoa butter or polyethylene glycol.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerin or sucrose and acacia.

Preferred unit dosage formulations are those containing an effective dose, an hereinbelow recited, or an appropriate fraction thereof, of the active ingredient.

It should be understood that in addition to the ingredients particularly mentioned above, the formulations of this invention may include other agents conventional in the art having regard to the type of formulation in

question, for example those suitable for oral administration may include flavouring agents.

Tablets or other forms of presentation provided in discrete units may conveniently contain an amount of compound of the invention which is effective at such dosage or as a multiple of the same, for instance, units containing 5mg to 500mg, usually around 10mg to 250mg.

The compounds of the invention are preferably used to treat CNS disorders or diseases by oral administration or injection (intraparenteral or subcutaneous). The precise amount of compound administered to a patient will be the responsibility of the attendant physician. However the dose employed will depend on a number of factors, including the age and sex of the patient, the precise disorder being treated, and its severity. Thus for example when treating a patient with epilepsy the dose range is likely to be significantly lower than when treating a patient after stroke to alleviate cerebral ischaemic damage. Also the route of administration is likely to vary depending on the condition and its severity.

The compounds of the invention may be administered orally or via injection at a dose of from 0.1 to 30mg/kg per day. The dose range for adult humans is generally from 8 to 2,400 mg/day and preferably 35 to 1,050 mg/day. As certain compounds of the invention are long acting, it may be advantageous to administer an initial dose of 70 to 2,400mg the first day then a lower dose of 20 to 1,200mg on subsequent days.

Long acting compounds in the clinic are advantageous because they are easier to manage. In the chronic situation, they may be administered without infusion and there is the minimum of direct medical intervention; also in acute conditions, patient compliance is encouraged by minimising daily dosing. Conversely, short acting compounds permit the clinician to control the pharmacological effect of the compound with great precision, since such compounds will be cleared from the central nervous system rapidly.

Compounds of the present invention may be made in any manner known to make analogous compounds known in the art (e.g. JACS vol 73 (1951) 3763-70).

Compounds 1 to 35 according to the invention which are set out above all conform to formula (I) with the substituents $R^1$ to $R^8$ taking appropriate meanings that are apparent from the compound names. In the following process description the term "compound of formula (I)" refers to one of the compounds 1 to 35 defined above or one of the four groups of compound of formula (I) set out above.

According to a first general process (A), compounds of formula (I) or an acid addition salt thereof, may be prepared by reacting an appropriate compound of formula (II)

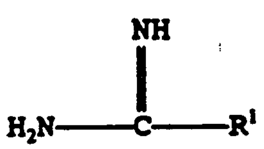

(II)

or a salt thereof, with an appropriate compound of formula (III)

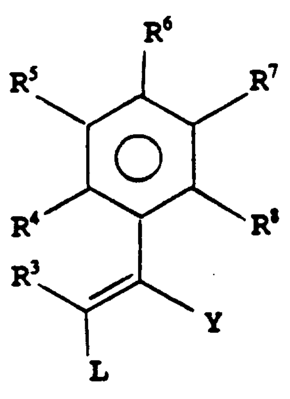

(III)

in which $R^1$ and $R^3$ to $R^8$ take the appropriate meanings for the compound of formula (I);
Y is cyano, carboxy, carbonyl, or alkoxycarbonyl; and L is a leaving group;
either or both of the compounds of formula (II) and (III) optionally being used in the form of a protected derivative thereof;

followed if necessary by removal of any protecting groups present.

Examples of suitable leaving groups include alkoxy e.g. $C_{1-4}$alkoxy, alkylthio, e.g. $C_{1-4}$alkylthio, halo, or optionally substituted amino, e.g. a group of formula $NR^9R^{10}$ where $R^9$ and $R^{10}$ are the same or different and are selected from hydrogen, alkyl, aryl and arylalkyl or $R^9$ and $R^{10}$, together with the nitrogen atom to which they are attached, form a heterocyclic ring optionally substituted by one or more alkyl groups and optionally containing a further heteroatom. Preferably in formula (III), L is alkylthio, or a group $NR^9R^{10}$, for example anilino, morpholino, $C_{1-4}$alkylamino or benzylamino.

Preferably the reaction of the compounds of formula (II) and (III) is carried out in a non-aqueous solvent, for example an alkanol, e.g. ethanol, at elevated temperatures (e.g. from 50 to 110°C). The compound of formula (II) is generally used in the form of a salt such as the hydroiodide salt, in which case the reaction is preferably carried out in the presence of a base, such as an alkanoxide, for example sodium ethoxide, preferably under reflux. Where the compound of formula (II) is used in the form of the free base, the addition of further base may be unnecessary.

According to a second general process (B), a compound of formula (I) or an acid addition salt thereof, may be made by the reaction of a compound of formula (II) or a salt thereof with a compound of formula (IV)

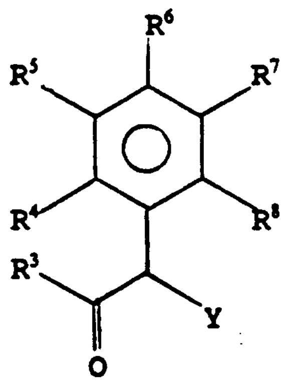

(IV)

in which $R^1$ and $R^3$ to $R^8$ take the appropriate meanings for the compound of formula (I); and Y is as defined in general process (A);
either or both of the compounds of formulae (II) and (IV) optionally being used in the form of a protected derivative thereof;
followed if necessary by removal of any protecting groups present.

The reaction preferably takes place in a non-aqueous solvent, e.g. an alkanol, such as ethanol, and at elevated temperatures, preferably under reflux.

According to a third general process (C), compounds of formula (I) or an acid addition salt thereof, may be made by the reaction of a compound of formula (II) or a salt thereof with a compound of formula (V)

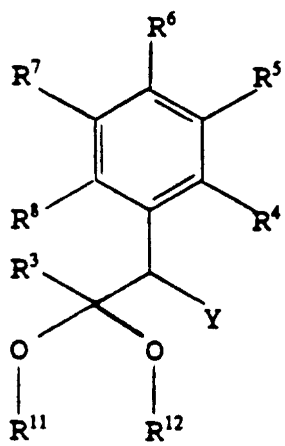

(V)

in which $R^1$ and $R^3$ to $R^8$ take the appropriate meanings for the compound of formula (I);
Y is as defined in general process (A);
and $R^{11}$ and $R^{12}$ are each alkyl or taken together form a group $(CR_2)_n$ where n is 2 to 4 and each R is independently hydrogen or alkyl, either or both of the compounds (II) and (V) optionally being used in the form of a protected derivative thereof;
followed if necessary by removal of any protecting groups present.

The reaction may be carried out in a non-aqueous solvent, e.g. ethanol, under reflux. The compound of formula (II) is generally used in the form of a salt such as the hydroiodide salt, in which case the reaction is preferably carried out in the presence of a base such as an alkanoxide, for example sodium ethoxide. Where the compound of formula (II) is used in the form of the free base, the addition of further base may be unnecessary.

According to a fourth general process (D), compounds of formula (I) may be prepared by dehydrogenation of the corresponding dihydropyrimidine or a protected derivative thereof, followed if necessary by removal of any protecting groups present. Dehydrogenation may be carried out under standard conditions, for example as described in J. Chem. Soc. 1956, 1019.

According to a fifth general process (E), compounds of formula (I) may be prepared by an interconversion reaction in which one compound of formula (I) is converted into another compound of formula (I).

An example of such an interconversion reaction is that a compound of formula (I) in which $R^4$ and/or $R^6$ is amino can be prepared by reduction of the corresponding compound in which $R^4$ and/or $R^6$ is nitro. Standard reduction conditions can be used, for platinum oxide catalyst, acetic acid and hydrogen gas.

Compounds in which a substituent, for example $R^4$ and/or $R^6$ is amino can be converted to compounds with other substituents $R^4$ and/or $R^6$, for example via the diazonium salt.

Compounds in which a substituent, for example $R^3$, is alkyl can be converted into the corresponding compound in which the substituent is a perhaloalkyl or halogenated alkyl moiety by reaction with the appropriate halogen or N-halo succinimide in a suitable solvent such as acetic acid.

A compound of formula (I) in which a substituent, for example $R^4$ and/or $R^6$ in nitro can be prepared by nitration of the corresponding compound in which $R^4$ and/or $R^6$ is hydrogen. The reaction can be carried out using standard nitration conditions.

It will be appreciated that other interconversions may be effected as required by those skilled in the art using standard methodologies.

Compounds of formula (I) can be isolated as the free base or as an acid addition salt. The compound of formula (I) in the form of the free base can be converted into an acid addition salt by standard methods, for example reacting the free base with the appropriate acid in a suitable solvent. Similarly one acid addition salt can be converted into another acid addition salt by standard methods.

Compounds of formula (II) can be made by known methods (see for example J. Amer. Chem. Soc. 1951, 73, 3763-3770) for example by the reaction of a compound of formula (IV) with diazomethane or with alkylorthoesters (J. Amer. Chem. Soc., 1952, 74, 1310-1313) or by condensation with an amine. Compounds of formula (IV) may themselves be made by known methods (J. Amer. Chem. Soc., 1951, 73, 3763-3770).

Compounds of formula (III) in which $R^1$ is piperazinyl or alkyl piperazinyl can also be made by standard methods, for example by reaction of a compound of formula (III) in which $R^1$ is alkylthio with an appropriate amine, e.g. N-methylpiperazine. The reaction is preferably carried out at room temperature in a suitable solvent such as water.

Dihydropyrimidines corresponding to the pyrimidines of formula (I) can be prepared by reaction of a compound of formula (III) in which L is hydrogen with a compound of formula (II).

The invention is illustrated by the following examples.

Example 1

2,4-Diamino-5-(3,5-dichlorophenyl)-6-trifluoromethylpyrimidine

(i) 3,5-Dichlorophenylacetonitrile

A mixture of 3,5-dichlorobenzyl alcohol (Aldrich, 25g), thionyl chloride (100ml) and DMF (0.5ml) was stirred and refluxed for 4 hours. After cooling the mixture was concentrated in vacuo, the residue was taken up in ether, washed with saturated aqueous $NaHCO_3$ and brine, dried ($MgSO_4$) and concentrated in vacuo to give 3,5-dichlorobenzylchloride as a light yellow solid, which was used without further purification, 28g, mp. 32-36 °C.

To a vigorously stirred solution of 3,5-dichlorobenzylchloride (28g) in dichloromethane (150ml) was added a mixture of KCN (27.5g) and tetrabutylammonium hydrogen sulphate (2.38g) in water (110ml). After stirring at room temperature for 22 hours, the mixture was diluted with dichloromethane, the organic phase washed with water and concentrated in vacuo to leave an oil. Filtration through silica with toluene followed by concentration then trituration with hexane gave the desired product as a colourless solid. 15.8g, mp. 31-32 ° C.

(ii) 2,4-Diamino-5-(3,5-dichlorophenyl)-6-trifluoromethylpyrimidine

To a stirred solution of NaOEt (from 2.2g sodium) in dry ethanol (40ml) at reflux, was added dropwise a mixture of 3,5-dichlorophenylacetonitrile (14.72g) and ethyl trifluoro acetate (Aldrich, 26.26g). After stirring at reflux for 4 hours, the mixture was cooled on ice. The yellow solid obtained after evaporating the ethanol was redissolved in water (60ml) and washed twice with diethyl ether. The aqueous layer was chilled, acidified with 1N hydrochloric acid, and extracted with diethyl ether 3 times. The combined extracts were washed with water and concentrated to give an oil. Trituration with hexane gave the 2-(3,5-dichlorophenyl)-3-oxo-3-trifluoromethyl propionitrile as a colourless solid (20g) and this was azetroped with toluene (x5).

To a chilled suspension of the acyl acetonitrile (1g) in hexane (10ml) was added in portions an excess of a cold solution of diazomethane in ether. After stirring overnight in room temperature, excess diazomethane was removed in vacuo to leave the crude enol ether. To a stirred solution of NaOEt (from 0.12g sodium) in ethanol (25ml) was added guanidine hydrochloride (0.5g). After 30 minutes of stirring at room temperature and ethanolic solution of the crude enol ether (prepared above) was added and then stirred under reflux for 5 hours. After cooling, the solvent was evaporated in vacuo. The solid obtained (1.28g) was chromatographed (silica; 1:1, MeOH:$CHCl_3$) to give the required product (0.74g) as a white solid. mp. 247-248 ° C.

H NMR DATA:
Solvent : DMSO
Assignment (δ) : 7.6(s,1H), 7.25(s,2H), 6.6(br.s,2H), 6.4(br.s,2H).

Example 2

2,4-Diamino-6-methyl-5-(2,3,6-trichlorophenyl)pyrimidine

(i) 2-(2,3,6-trichlorophenyl)-3-oxobutyronitrile

To a stirred solution of NaOEt (from 2.72g sodium) in ethanol (50ml) at reflux, was added dropwise a mixture of 2,3,6-trichlorophenylacetonitrile (Lancaster synthesis, 20g) and ethyl acetate (21.5g).

After stirring at reflux for four hours, the mixture was cooled on ice.

The solid obtained after evaporating the ethanol in vacuo was dissolved in water (100ml) and washed twice with ether. The aqueous layer was chilled and acidified. The product was extracted with ether. After removing the ether from the extracts a white solid was obtained.

(ii) 2,4-Diamino-6-methyl-5-(2,3,6-trichlorophenyl) pyrimidine

To a suspension of the acyl nitrile (1g) in ether was added in portions an excess of a solution of diazomethane in ether. After stirring for 2 hours at room temperature, the ether was removed in vacuo leaving behind the enol ether.

To a stirred solution of NaOEt (from 0.32g sodium) in ethanol (30ml) was added guanidine hydrochloride (0.66g). After 30 minutes a solution of the above enol ether (1g) in ethanol (5ml) was added and then stirred and refluxed for 5 hours. After cooling, the solvent was evaporated in vacuo. The brown solid obtained was chromatographed twice (Silica; 1:9, methanol : chloroform) and (silica; 1:1, ethyl acetate : chloroform) to give the required product. mp. sublime at 240 ° C.

NMR analysis
Solvent : deuterated methanol
Assignment (δ) : 8.4(m,2H), 6.7(br.s,2H), 6.6(br.s,2H), 2.5(s,3H).

Example 3

2,4-Diamino-5-(4-amino-2,3,5-trichlorophenyl)pyrimidine

(i) 2,3,5-trichlorobenzylalcohol

To a solution of 2,3,5-trichlorobenzaldehyde (Aldrich, 50gms) in ethanol (1.01) at room temperature was added $NaBH_4$ (7.00gms) and the resulting mixture stirred for 3.5 hours. The reaction was quenched with water, and the solvent evaporated in vacuo before partitioning the residue between $CHCl_3$ and saturated $NaHCO_3$ solution. The organic phase was washed with brine, dried over $MgSO_4$, filtered and the solvent evaporated in vacuo to leave a white solid. 43.00gms, mp. 90-93 °C.

(ii) 2,3,5-trichlorobenzyl bromide

To a solution of the alcohol in benzene (400ml) under $N_2$ was added $PBr_3$ (126.58gms), and the mixture stirred at 55-60 °C for 3.5 hours. After cooling, the mixture was poured onto crushed ice (2l) and the benzene layer separated. The aqueous phase was washed with benzene (x3) and the combined benzene extracts washed with saturated $NaHCO_3$ solution and water, dried over $MgSO_4$, filtered and the solvent evaporated to leave a brownish liquid which solidified on standing, 37.53gms, mp. 40-42 °C.

(iii) 2,3,5-trichlorophenylacetonitrile

The bromide was suspended in DMF (130ml)/water(86.67ml) at 0 °C and KCN(12.99gms) added in portions. After stirring at 30-35 °C for 3 hours, the suspension was diluted with water and extracted with $Et_2O$. The combined ether extracts were washed with water, dried over $MgSO_4$, filtered and the solvent evaporated in vacuo. Chromatography on silica gel eluting with hexane to 20% ether-hexane gave the desired product as a white solid, 18.52gms, mp. 60-62 °C.

(iv) 2-(2,3,5-trichlorophenyl)-3-oxo-propionitrile, sodium salt

To a solution of NaOEt (from 0.803g of sodium) in ethanol (55ml) cooled in ice, under nitrogen, was added 2,3,5-trichlorophenyl acetonitrile. Ethyl formate (5.1ml) was added and the mixture was stirred at room temperature overnight. After stirring for a further 2.5 hours at 50 °C, the mixture was cooled and filtered. The filtrate was evaporated, and the residue was triturated with diethyl ether, filtered and dried (6.82g).

(v) 2-(2,3,5-trichlorophenyl)-3-methoxy-acrylonitrile

The above solid was dissolved in DMF (36ml) and methyl iodide (2ml) was added. The reaction vessel was sealed before stirring the contents at 40 °C for 3 hours. The solvent was then evaporated. The residue was partitioned between water and ethyl acetate. The organic phase was washed with water, dried ($MgSO_4$) and the solvent evaporated to give the crude product as a red-brown oil that solidified on standing (5.04g).

(vi) 2,4-Diamino-5-(2,3,5-trichlorophenyl)pyrimidine

Guanidine hydrochloride (3.20g) was added to a solution of sodium ethoxide (from 848mg sodium) in ethanol (52ml). The resulting white suspension was stirred at room temperature for 10 minutes. The above enol ether was added and the resulting mixture stirred at reflux for 3.5 hours. After cooling, the suspension was filtered, and the filtrate evaporated to dryness in vacuo. Chromatography on silica gel eluting with $CHCl_3$ to 3% MeOH-$CHCl_3$ gave the desired product which was triturated with ether and dried in vacuo. Yield = 2.01g, mp. 246-249 °C.

(vii) 2,4-Diamino-5-(4-nitro-2,3,5-trichlorophenyl) pyrimidine

A portion (100mg) of the compound from (vi) was dissolved in concentrated sulphuric acid (2.5ml), potassium nitrate (25.8mg) added and the solution stirred for 3 hours. The solution was then poured onto ice and basified with 10 N NaOH. The product was extracted with ethyl acetate (x3), dried over $MgSO_4$, filtered and the solvent evaporated. Chromatography on silica gel eluting with ethyl acetate gave the desired

product, 40.7mg, mp. 293-295 ° C.

(viii) 2,4-Diamino-5-(4-amino-2,3,5-trichlorophenyl) pyrimidine

The compound from (vii) (350mg) was dissolved in glacial AcOH (20ml) platinum IV oxide (18mg) added and the mixture stirred for 1 hour under $H_2$ atmosphere. The mixture was filtered through Hyflo, the filtrate concentrated arid the residue neutralised with saturated sodium bicarbonate solution. The product was extracted with ethyl acetate (x3), dried over $MgSO_4$ and filtered and the solvent evaporated. Chromatography on silica gel eluting with 5% methanol/ethyl acetate gave the desired product, 260mg, mp. 284-286 ° C.
NMR Analysis
Solvent - DMSO-$d_6$
Assignment (δ) 7.55(s,1H), 7.45(s,1H), 6.1-5.9(br.d,4H,-2$NH_2$) 5.1-4.95(br.s,2H,-$NH_2$).

Example 4

4-Amino-2-(4-methyl-1-piperazinyl)-6-methyl-5-(3,4,5-trichlorophenyl)pyrimidine

(i) 3,4,5-Trichlorobenzoic acid

To a stirred solution of 4-amino-3,5-dichlorobenzoic acid (Lancaster synthesis; 28g) in a mixture of concentrated sulphuric acid (200ml) and acetic acid (140ml) was added sodium nitrite (11.28g) in portions over 30 minutes. The mixture was stirred at room temperature for 5 hours then added slowly to a vigorously stirred suspension of freshly prepared cuprous chloride (20g) in concentrated hydrochloric acid (360ml). After standing overnight the precipitate was filtered, washed with water and dried in vacuo at 100 ° C to provide the title compound. 28.6g, mp. 197-199 ° C.

(ii) 3,4,5-Trichlorobenzylalcohol

To a stirred solution of 3,4,5-trichlorobenzoic acid (11.7g) in a mixture of dry THF (50ml) and trimethylborate (25ml) under $N_2$ at room temperature was added borane-dimethylsulphide complex (ca. 10M; 7.5ml) dropwise over 45 min. After stirring for a further 4 hours methanol (25ml) was added dropwise over 45 minutes. The mixture was stirred for a further 30 minutes then evaporated in vacuo. The residue was dissolved in ether, washed with 2M sodium hydroxide and brine, dried ($MgSO_4$) and concentrated in vacuo. Trituration of the residue with hexane gave the title compound, 9.9g, mp. 106-108 ° C.

(iii) 3,4,5-Trichlorophenylacetonitrile

A mixture of 3,4,5-trichlorobenzyl alcohol (17.4g), thionyl chloride (75ml) and DMF (0.5ml) was stirred and refluxed for 4 hours. After cooling the mixture was concentrated in vacuo, the residue taken up in ether, washed with saturated aqueous $NaHCO_3$ and brine, dried ($MgSO_4$) and concentrated in vacuo to give 3,4,5-dichlorobenzylchloride (l9g), which was used without further purification.

To a vigorously stirred solution of 3,4,5-trichlorobenzylchloride (l9g) in dichloromethane (85ml) was added a mixture of KCN (16.07g) and tetrabutylammonium hydrogen sulphate (1.38g) in water (85ml). After stirring at room temperature for 21 hours the mixture was diluted with dichloromethane, the organic phase washed with water and concentrated in vacuo to leave an oil. Filtration through silica with toluene followed by concentration then trituration with hexane gave the title compound. 11.9g, mp. 56-58 ° C.

(iv) 4-Amino-2-(4-methylpiperazinyl)-6-methyl-(3,4,5-trichlorophenyl)pyrimidine.

To a stirred solution of NaOEt (from 0.42g sodium), in ethanol (25ml) at reflux, was added over 5 minutes a mixture of 3,4,5-trichlorophenylacetonitrile (3.31g) and ethyl acetate (2.03g) in dry dimethoxyethane (10ml). After stirring at reflux for 4 hours the mixture was cooled in ice, acidified with acetic acid, poured into cold water and concentrated to give an oil. Trituration with hexane gave 2-(3,4,5-trichlorophenyl)-3-oxobutyronitrile as a colourless solid (1.8g) which was used without further purification.

To a solution of 2-(3,4,5-trichlorophenyl)-3-oxobutyronitrile (3.41g) in ether (100ml) was added in portions as excess of a solution of diazomethane in ether. After stirring for 2 hours at room temperature the solution was concentrated in vacuo to give 2-(3,4,5-trichlorophenyl)-3-methoxybut-2-enonitrile (3.6g) which

was used without further purification.

To a stirred solution of NaOEt (from 0.26g sodium) in ethanol (10ml) was added N-methylpiperazinoformamidine hydroiodide produced in the manner outlined below (Example 10(i)) (2.6g). After 10 minutes a solution of 2-(3,4,5-trichlorophenyl)-3-methoxybut-2-enonitrile (1.8g) in dry ethanol (15ml) was added and the mixture stirred and refluxed for 4 1/2 hours. After cooling the solvent was evaporated in vacuo and the residue shaken with 2M NaOH (50ml). The solid was filtered off, washed with water, dissolved in chloroform, washed with water and concentrated in vacuo. The residue was triturated with hexane to give a solid (1.2g) which was further purified by chromatography (silica; 1:19 to 1:9 MeOH:$CHCl_3$) to give the title compound. 0.92g, mp. 222-223 °C.

NMR Analysis:
δH (DMSO); 1.9(3H,s), 2.2(3H,s), 2.3(4H,m), 3.65(4H,m), 5.95(2H,br.s.), 7.45(2H,s).

Example 5

2,4-Diamino-5-(3,4,5-trichlorophenyl)-6-methylpyrimidine

To a solution of sodium ethoxide (from 0.18g sodium) in dry ethanol (10ml) at room temperature was added guanidine hydrochloride (0.62g). After 15 minutes a solution of 2-(3,4,5-trichlorophenyl)-3-methoxybut-2-enonitrile (Example 4(iv)) [QUANTITY?] in ethanol (25ml) was added and the mixture stirred at reflux for 4 hours. After cooling the solvent was evaporated in vacuo. The residue was suspended in 2M NaOH (50ml), filtered, washed with water, dried in air and recrystallised from ethanol to give the title compound. 0.2g, mp. 280-281 °C.

NMR Analysis:
δH (DMSO); 1.9(3H,s), 5.85(2H,br.s.), 5.95(2H,br.s.), 7.45(2H,s).

Example 6

4-Amino-2-diethylamino-5-(2,3,5-trichlorophenyl)pyrimidine

To a solution of NaOEt (from 52.6mg of sodium) in ethanol (5ml) was added 1,1-diethylguanidine hydrochloride (Bader, 0.288g). After stirring for 10 minutes the adduct of Example 3(v) (0.25g) was added and the mixture was stirred at reflux for 4 hours. The mixture was left standing at room temperature overnight and then filtered. The filtrate was concentrated and purified by chromatography on $SiO_2$, eluting with $CHCl_3$ to give the desired product, 0.173g, 105-107 °C.

NMR Analysis
δH ($CDCl_3$) 1.12-1.3(6H,t), 3.52-3.68(4H,q), 4.35-4.58(2H,br.s), 7.23(1H,d), 7.48(1H,d), 7.82(1H,s).

Example 7

4-Amino-2-(2-iso-propylamino)-5-(2,3,5-trichlorophenyl) pyrimidine

To a solution of NaOEt (from 52.6mg of sodium) in ethanol (5ml) was added 1-isopropylguanidine sulphate (Bader, 0.379g). After stirring for 10 minutes, the adduct of Example 3(v), (0.11g), was added and the mixture was stirred at reflux for 4 hours. The mixture was left standing at room temperature overnight and then filtered. The filtrate was concentrated and the residue was purified by chromatography on $SiO_2$, eluting with $CHCl_3$ to give the desired product, 86.9mg, mp. 182-184 °C.

NMR Analysis:
δH ($CDCl_3$) 1.18-1.37(6H,d), 4.03-4.25(1H,m), 4.45-4.65(2H,br.s.), 4.72-4.88(1H,br.d.), 7.23(1H,d), 7.5(1H,d), 7.77(1H,s).

Example 8

4-Amino-5-(3,5-dichlorophenyl)-2-(4-methyl-1-piperazinyl)pyrimidine

(i) 2-(3,5-Dichlorophenyl)-3-oxopropionitrile

To a stirred solution of NaOEt (from 0.6g sodium) in ethanol (10ml) at reflux, was added dropwise a mixture of 3,5-dichlorophenylacetonitrile (Example 1(i), 4g) and ethyl formate (Aldrich 3.8g). After stirring for

four hours, the mixture was cooled.

The solid obtained after evaporating the ethanol was dissolved in water. This solution was washed twice with ether. After chilling, this solution was acidified with hydrochloric acid. The product was extracted with ether. A white solid was obtained after evaporating the solvent (4.35g crude).

(ii) 4-Amino-5-(3,5-dichlorophenyl)-2-(4-methyl-1-piperazinyl) pyrimidine

To a solution of 2-(3,5-dichlorophenyl)-3-oxopropionitrile (4.35g) in ether (100ml) was added in portions an excess of a cold solution of diazomethane in ether.

After stirring for 2 hours at room temperature the solvent was removed leaving behind the enol ether (4.07g).

To a stirred solution of NaOEt (from 0.5g sodium) in ethanol (20ml) was added N-methylpiperazinoformamidine hydroiodide (3.5g). After 30 minutes, a solution of the above enol ether (4.07) in ethanol (15ml) was added and then stirred and refluxed for 5 hours. After cooling, the solvent was evaporated in vacuo. This material was purified by recrystallisation from chloroform to give the desired product 1.5g, mp. 161.5-162 °C.

NMR analysis (δ)

Solvent $CDCl_3$

Assignment - 7.85(s,1H), 7.35(m,3H), 4.8(br.s,2H), 3.85(t,4H), 2.45(t,4H), 2.35(s,3H).

Example 9

2,4-Diamino-5-(4-iodo-2,3,5-trichlorophenyl)pyrimidine

The compound of Example 3 (0.305g) was dissolved in concentrated HCl (0.8ml) and water (1.2ml). The mixture was cooled in an ice bath before a solution of sodium nitrite (0.096g in 0.5ml $H_20$) was added. The mixture was stirred at room temperature for 2 hours before potassium iodide solution (0.305g in 1.2ml $H_20$) was added to the cooled mixture. The reaction was stirred at 90 °C for 2 hours.

The mixture was then basified with saturated sodium bicarbonate solution, the product extracted with ethyl acetate, dried over $MgSO_4$, filtered and the solvent evaporated. Chromatography on silica gel eluting with dichloromethane/ethanol (15/1) gave the desired product, 88mg, mp. 290 °C (decomposition).

NMR analysis (δ)

DMSO-$d_6$

7.95(s,1H), 7.4(s,1H), 6.15-5.95(br.s,4H,-$2NH_2$)

Example 10

4-Amino-2-(4-methyl-4-oxidepiperazin-1-yl)-5-(2,3,5-trichlorophenyl)-6-trifluoromethylpyrimidine

(i) N-methylpiperazinoformamidine hydroiodide

Thiourea (10.8g) was dissolved in acetone (250ml) at 50 °C. Iodomethane (10ml) was added and the reaction was stirred at 50 °C for 4 hours. After cooling, the solution was diluted with ether (1 litre) and the methiodide salt was filtered, washed with ether and dried in vacuo, 29.2g, 113-115 °C. The methiodide salt (5g) was dissolved in water (30ml) and N-methylpiperazine was added. The solution was stirred, with nitrogen bubbled through, at room temperature for 24 hours. The solution was concentrated in vacuo. The residue was slurried with ethanol, filtered and dried in vacuo, 4.98g, mp. 230-242 °C.

(ii) 2-(2,3,5-trichlorophenyl)-4,4,4-trifluoro-3-oxobutyronitrile

To a solution of NaOEt (from 1.04gms Na) in EtOH (60ml) at room temperature under $N_2$ was added 2,3,5-trichlorophenyl acetonitrile (Example 3(iii), 8.40gms) followed by ethyl trifluoroacetate (6.57gms) and the mixture stirred at reflux for 5 hours. After cooling, the solvent was removed in vacuo and the residue dissolved in water. The aqueous phase was washed with $Et_2O$ (discarded), acidified with $H_2SO_4$ and extracted with $Et_20$. The combined $Et_20$ extracts were washed with water, dried over $MgSO_4$, filtered and the solvent evaporated in vacuo to leave an oil. This was triturated with petroleum ether, and the solid filtered off and dried. The solid was azeotroped with toluene (x5), 4.89gms, mp. 160-163 °C.

(iii) 2-(2,3,5-trichlorophenyl)-4,4,4-trifluoro-3-methoxybut-2-enonitrile

To a solution of the trifluoromethyl ketone in $Et_2O$ (39.62ml) at room temperature was added diazomethane (from 8.55gms Diazald) in $Et_2O$ (79.62ml), and the resulting mixture left to stand at room temperature overnight. Excess diazomethane was then removed in vacuo into AcOH, and the residue was dissolved in $Et_2O$, dried over $MgSO_4$, filtered and the solvent evaporated in vacuo to leave a brownish oil, 5.20gms.

(iv) 4-Amino-2-(4-methylpiperazin-1-yl)-5-(2,3,5-trichlorophenyl)-6-trifluoromethylpyrimidine

To a solution of NaOEt (from 0.144g of Na) in EtOH (12.5ml) was added N-methylpiperazinoformamidine hydroiodide (1.39g). After stirring for 10 minutes at room temperature a solution of the above intermediate (0.85g) in EtOH (2.5ml) was added and the resulting mixture was stirred at reflux for 4.5 hours. After cooling, the suspension was filtered, and the filtrate was evaporated to dryness in vacuo. Chromatography on silica, eluting with $CHCl_3$ -4% MeOH/$CHCl_3$, gave the desired product which was triturated with petroleum ether (b.p. 40-60 °C) and dried in vacuo. 0.56g, mp. 127-129 °C.

(v) 4-Amino-2-(4-methyl-4-oxidepiperazinyl)-5-(2,3,5-trichlorophenyl)-6-trifluoromethylpyrimidine

A solution of 3-chloroperoxybenzoic acid (0.225g) in chloroform (12ml) was added dropwise to the solution of the product of (iv) (0.33g) in chloroform (12ml) maintaining the temperature below 5 °C. The resulting solution was stirred at room temperature for 18 hours. The reaction mixture was washed with 1N sodium hydroxide (x3), water (x2), dried over $MgSO_4$, filtered and the filtrate evaporated under reduced-pressure to give a cream solid, 0.150g, mp. 185-188 °C.
NMR analysis
Solvent - DMSO-$d_6$
$\delta$ 2.90(d,2H), $\delta$ 3.0(s,3H), $\delta$ 3.30(t,2H), $\delta$ 3.60(t,2H), $\delta$ 4.40(d,2H), $\delta$ 6.10(s,br,2H), $\delta$ 7.40(s,1H), $\delta$ 7.90(s,1H)

Example 11

4-Amino-5-(3,5-dichlorophenyl)-6-methoxymethyl-2-(4-methyl-1-piperazinyl)pyrimidine bis methanesulphonate

To a stirred solution of NaOEt (from 0.7g sodium) in ethanol (15ml) at reflux, was added dropwise a mixture of 3,5-dichlorophenylacetonitrile (Example 1(i), 5g) and ethyl methoxyacetate (Aldrich, 14g). After stirring at reflux for 4 hours, the mixture was cooled on ice. The solid obtained after evaporating the solvent was dissolved in water (25ml). This solution was extracted twice with diethyl ether.

The aqueous layer was chilled and acidified with hydrochloric acid. The product was extracted with diethyl ether and the combined extracts washed with water. Evaporating the solvent gave 2-(3,5-dichlorophenyl)-4-methoxy-3-oxobutyronitrile as a brown solid (5.9g).

To a suspension of the acyl acetonitrile (5.9g) in ether (5.0ml) was added in portions an excess of a cold solution of diazomethane in ether. After stirring for 2 hours at room temperature, the solution was concentrated to give the enol ether.

To a stirred solution of NaOEt (from 0.5g sodium) in ethanol (40ml) was added N-methylpiperazinoformamidine hydroiodide (6g). After 30 minutes a solution of the above enol ether (4.69g) in ethanol (20ml) was added and then stirred and refluxed for 5 hours. After cooling, the solvent was evaporated in vacuo. The brown solid obtained was chromatographed (silica, 1:9, MeOH;$CHCl_3$) to give brown oil (0.69g).

To a solution of this material (0.69g) in ethanol (20ml), was added dropwise a solution of methanesulphonic acid (0.21g) in ethanol (2ml). This mixture was stirred at room temperature for 3 hours. After standing overnight the precipitate that formed was filtered off and washed with fresh ethanol. After triturating with ether and drying, the desired product was obtained as a colourless bis methanesulphonate salt, 0.27g, mp. decomposes above 210 °C.
NMR analysis ($\delta$)
Solvent - $CDCl_3$
Assignment - 7.3(m,3H), 4.5(br.s,2H), 4.05(s,2H), 3.85(t,4H), 3.3(s,3H), 2.45(t,4H), 2.35(s,3H).

Example 12

2,4-Diamino-5[(4-(N'N'-dimethylsulphamoyl)-2,3,5-trichlorophenyl]pyrimidine

The compound of example 3 (0.305g) was dissolved in concentrated HCl (0.8ml) and water (1.2ml). The mixture was cooled in an ice bath before a solution of sodium nitrite (0.096g in 0.5ml $H_20$) was added. The mixture was then stirred at room temperature for 2 hours. Cupric chloride (49.7mg) and 5.14M $SO_2$/AcOH (0.97ml) were added to the cooled reaction mixture before being stirred at 5°C for 2 hours. The solid (250mg) produced was filtered and washed with water.

The solid was dissolved in THF (4ml) before 25/30% w/v aqueous dimethylamine (4ml) was added. The reaction mixture was stirred at room temperature for 2 hours before being diluted with water (10ml). The product was extracted with ethyl acetate, dried over $MgSO_4$, filtered and solvent evaporated. Chromatography on silica eluting with chloroform/methanol (20/1) gave the desired product, 38mg, mp. 201°C (decomposition).

NMR analysis ($\delta$)
Solvent - DMSO-$d_6$
8.10(s,1H), 7.30(s,1H), 6.05-6.00(br.s,2H,-$NH_2$), 5.95-5.85(br.s,2H,-$NH_2$), 2.7(s,6H).

Example 13

2-Amino-5-(2,3-dichlorophenyl)-4-(1-piperidinyl)pyrimidine

(i) 2,3-Dichlorophenylacetic acid

Concentrated hydrochloric acid (100ml) was poured onto crushed ice (150ml), the solution added to 2,3-dichlorophenylacetonitrile (30.6gms), and the mixture refluxed for 3 hours. After cooling, the mixture was diluted with water (500ml), extracted with EtOAc (600ml) and the organic phase washed with brine before drying over $MgSO_4$. Evaporation of the solvent left a white solid, 31.3gms.

(ii) Ethyl 2,3-dichlorophenylacetate

To a suspension of the acid in ethanol (200ml) was added concentrated $H_2SO_4$ (1ml) and the mixture stirred at reflux for 3 hours. After cooling, the solvent was evaporated and the residue treated with concentrated $NH_40H$ (3ml) in water (50ml). The organic phase was extracted into $CH_2Cl_2$, dried over $MgSO_4$, and the solvent evaporated to leave a clear liquid, 19.88gms.

(iii) Ethyl-2-(2,3-dichlorophenyl)-3-N-morpholino-acrylate

To a mixture of the ester, morpholine (40.7gms) and ethyl orthoformate (69.24gms) was added acetic anhydride (0.5ml) and the resulting pale yellow solution stirred at reflux for 3 hours. After cooling, the mixture was concentrated in vacuo. A white precipitate started to form, and this was filtered off before further concentrating the filtrate to give a brownish clear oil. Standing overnight in vacuo gave a yellow solid, 34.34gms.

(iv) 5-(2,3-dichlorophenyl)isocytosine

To the ester of (iii) was added guanidine hydrochloride (26.6gms) slurried in sodium 2-methoxyethoxide (from 6.6gms of Na) in 2-methoxyethanol (150ml), and the mixture stirred at reflux overnight. After cooling, the mixture was concentrated in vacuo, diluted with water (100ml) and then washed with $Et_2O$ (200ml). The aqueous phase was acidified with AcOH, and the precipitate filtered off and washed with EtOH and then $Et_2O$ before drying in vacuo, 13.48gms.

(v) $N^1$-[4-chloro-5-(2,3-dichlorophenyl)-2-pyrimidinyl]-$N^2$,$N^2$-dimethylformamidine

To a mixture of the isocytosine of (iv) (14.4gms) in $CH_2Cl_2$ (200ml) was added dropwise during 30 minutes fresh Vilsmeier-Haack reagent (from 2.75 equivalents of $SOCl_2$ and 2.58 equivalents of DMF), and the mixture refluxed for 6 hours. After cooling, 1N NaOH (250ml) was added slowly. The aqueous phase was washed with $CH_2Cl_2$ and the combined organic extracts washed with brine before drying over $MgSO_4$.

Evaporation of the solvent and chromatography on $SiO_2$, eluting with EtOAc gave 13.6gms, mp. 113-115 °C.

(vi) 2-Amino-4-chloro-5-(2,3-dichlorophenyl)pyrimidine

To the formamidine in EtOH (50ml) was added ethanolic $MeNH_2$ (8 equiv.) in EtOH (50ml), and the mixture sealed in a Parr reaction vessel before stirring at room temperature for 5 hours. The reaction mixture was then concentrated in vacuo, the residue mixed with 1N NaOH (75ml), filtered, washed with water and dried in vacuo, 11.2gms, mp. 228-30 °C.

(vii) 2-Amino-5-(2,3-dichlorophenyl)-4-(1-piperidinyl)pyrimidine

2-Amino-4-chloro-5-(2,3-dichlorophenyl)pyrimidine (0.1g) and piperidine (0.5ml) were stirred at 60 °C in a sealed vessel with ethanol (2ml) for 20 hours. The resulting mixture was evaporated to dryness and the crude residue purified by column chromatography on silica, eluting with chloroform. The product was slurried in saturated sodium bicarbonate solution, filtered and washed well with water. Yield 0.065g, mp. 187-9 °C.
NMR Analysis (δ)
Solvent - $CDCl_3$
Assignment - 7.70(s,1H), 7.40(m,1H), 7.20(m,2H), 4.80(br.s,2H), 3.25(br.m,4H), 1.30-1.80(br.m,6H).

Example 14

4-Amino-5-(3,5-dichlorophenyl)-2-(4-methyl-1-piperazinyl)-6-trifluoromethylpyrimidine

To a stirred solution of NaOEt (from 1.6g of sodium), in ethanol (30ml) at reflux, was added dropwise a mixture of 3,5-dichlorophenylacetonitrile (Example 1(i), 10g) and ethyl trifluoroacetate (Aldrich, 17.5g). After stirring at reflux for 4 hours, the mixture was cooled on ice. The yellow solid obtained after evaporating the ethanol was redissolved in water (50ml). This alkaline aqueous solution was extracted twice with diethyl ether.

The aqueous layer was chilled on an ice bath and acidified with hydrochloric acid. The product was extracted with diethyl ether 3 times. The combined extracts were washed with water and concentrated to give an oil. Trituration with hexane gave 2-(3,5-dichlorophenyl)-3-oxo-3-trifluoromethylpropionitrile as a colourless solid (12.0g).

To a chilled suspension of the acyl acetonitrile (1g) in hexane (70ml) were added in portions an excess of a cold solution of diazomethane in ether. After stirring for 2 hours at room temperature the solution was concentrated to give the enol ether.

To a stirred solution of NaOEt (from 0.25g sodium) in ethanol (30ml) was added N-methylpiperazinoformamidine hydroiodide (3g). After 30 minutes a solution of the above enol ether (2.3g) in ethanol (10ml) was added and then stirred and refluxed for 5 hours. After cooling, the solvent was evaporated in vacuo. The solid was chromatographed (silica, 1:9 MeOH:$CHCl_3$) to give the desired product as a colourless solid, 2.4g, mp. 159.5-160 °C.
NMR Analysis
Solvent $CDCl_3$
Assignment (δ) 7.25(m,3H), 4.65(br.s,2H), 3.85(t,4H), 2.45(t,4H), 2.35(s,3H).

Example 15

2,4-Diamino-5-(2,3,4,5-tetrachlorophenyl)pyrimidine

2,4-Diamino-5-(4-amino-2,3,5-trichlorophenyl)pyrimidine (Example 3, 0.3045g) was dissolved in hot glacial acetic acid (12.68ml) and to this was added a solution of sodium nitrite (0.097g) in 0.67ml of concentrated sulphuric acid. The mixture was stirred for 2 hours before being added to a solution of cuprous chloride (0.15g in 0.5ml concentrated hydrochloric acid) at 60 °C. The reaction mixture was allowed to cool, basified with sodium bicarbonate, extracted with ethyl acetate, dried with $MgSO_4$ and solvents evaporated. Chromatography on silica eluting with ethyl acetate/ethanol (20/1) gave the desired product. Yield: 150mg, mp. 314-317 °C.
'H NMR Analysis (δ)
Solvent - DMSO-$d_6$

Assignment - 8.05(s,1H), 6.15-6.00(2 x s,4H,-$2NH_2$), 7.50(s,H).

Example 16

cis 4-Amino-2-(3,5-dimethyl-l-piperazinyl)-5-(2,3,5-trichlorophenyl)pyrimidine mesylate

(i) cis-3,5-dimethylpiperazinoformamidine hydroiodide

The methiodide salt of Example 10(i), (4.5g) was dissolved in water (30ml) and 2,6-dimethylpiperazine (Aldrich, 4.71g) was added. The solution was stirred, with nitrogen bubbled through, at room temperature for 24 hours. The solution was concentrated in vacuo. The residue was triturated with hot ether, filtered and dried in vacuo, 5.81g, 210-213 °C.

(ii) cis 4-Amino-2-(3,5-dimethyl-1-piperazinyl)-5-(2,3,5-trichlorophenyl)pyrimidine mesylate

To a solution of NaOEt (from 0.24g of sodium) in ethanol (30ml) was added cis-3,5-dimethylpiperazinoformamidine hydroiodide (2.43g). After stirring for 10 minutes, the adduct of Example 3(v) (1.5g) was added and the mixture was heated under reflux for 3 hours. The mixture was left standing at room temperature overnight and then filtered. The filtrate was concentrated and the residue was purified by chromatography on $SiO_2$, eluting with $CHCl_3$-8% MeOH/$CHCl_3$ to give the desired product, 1.64g.

The phenyl pyrimidine base (1.61g) was dissolved in absolute ethanol, cooled at 0 °C, and methanesulphonic acid (0.27ml) was added. After stirring at room temperature for 2 hours, the solvent was evaporated to dryness and the residue was triturated with $Et_2O$, filtered and dried. The residue was dissolved in water and freeze dried, 1.76g, 300-305 °C.

NMR Analysis:

δH (DMSO); 1.27(6H,d), 2.33(3H,s), 2.68-2.88(2H,br.t), 4.7-4.87(2H,br.d), 6.47(2H,br.s), 7.35(1H,d), 7.7(1H,s), 7.81(1H,d).

Example 17

4-Amino-2-(4-n-propyl-1-piperazinyl)-5-(2,3,5-trichloro phenyl)pyrimidine dimesylate

N-propylpiperazine dihydrobromide (Lancaster 5g) in water (15ml) was passed into an ion exchange column (IR410 OH form) (BDH) and eluted with water. The secondary amine positive element was concentrated, dissolved in diethylether, dried ($MgSO_4$) and evaporated to dryness to give N-propylpiperazine as a colourless oil (0.6g).

The piperazine was dissolved in water (10ml) and methylthiouronium iodide (Example 1(i)) added. The solution was stirred, with nitrogen bubbled through, at room temperature for 96 hours. The solution was concentrated in vacuo. The residue was slurried with acetone, filtered and dried in vacuo, 0.51g, mp. 174-176 °C.

To a solution of NaOEt (from 0.052g of sodium) in ethanol (5ml) was added N-propylpiperazinoformamidine hydroiodide (Example 10(i), 0.506g). After stirring for a further 10 minutes, the enol ether of Example 3(v) (0.226g) was added and the mixture was stirred at reflux for 5 hours. The mixture was concentrated, dissolved in chloroform and filtered. The filtrate was reconcentrated and the residue was purified by chromatography on $SiO_2$, eluting with 5% MeOH/$CHCl_3$ to give the title compound as the free base. 0.26g.

The free base (0.26g) was dissolved in $Et_20$ and cooled in an ice bath. Methanesulphonic acid (0.062g) was added and the reaction was stirred in the ice bath for 1 hour. The solid was filtered and dried in vacuo to give the title salt as a yellow amorphous solid, 0.21g, dec. 83 °C.

NMR Assignment (δ)

Solvent - DMSO

δ 0.95(t,3H), δ 1.75(m,2H), δ 2.35(s,6H), δ 3.10(d,br,2H), δ 3.40(t,br,4H), δ 3.60(d,br,2H), δ 4.60(d,2H), δ7.45-(d,1H), δ 7.90(s,1H), δ 7.95(d,1H).

Example 18

4-Amino-2-(4-methyl-4-oxide-1-piperazinyl)-5-(2,3,5-trichlorophenyl)pyrimidine

A solution of 3-chloroperoxybenzoic acid (0.34g) in chloroform (18ml) was added dropwise to the solution of 4-amino-2-(4-methyl-1-piperazinyl)-5-(2,3,5-trichlorophenyl)pyrimidine (0.43g) in chloroform (18ml) maintaining the temperature below 5°C. The resulting solution was stirred at room temperature for 18 hours. The reaction mixture was washed with 1N sodium hydroxide (x3), water (x2), dried over $MgSO_4$, filtered and the filtrate evaporated under reduced-pressure. The residue was triturated with water, filtered, suspended in water and freeze-dried to give a cream solid, 0.052g. mp. 180-183°C.
NMR Assignment ($\delta$)
Solvent - DMSO-$d_6$
$\delta$ 2.9(d,2H), $\delta$ 3.1(s,3H), $\delta$ 3.3(t,2H), $\delta$ 3.4(t,2H), $\delta$ 4.4(d,2H), 6.4(s,br,2H), $\delta$ 7.3(s,1H), $\delta$ 7.6(s,1H), $\delta$ 7.8(s,1H).

Example 19

2,4-Diamino-5-(3,5-dichloro-2-nitrophenyl)-6-methyl pyrimidine

2,4-Diamino-5-(3,5-dichlorophenyl)-6-methyl pyrimidine (0.377g) was dissolved in concentrated $H_2SO_4$ - (15ml) before potassium nitrate (0.15g) was added. The mixture was stirred at room temperature for 4 hours and then diluted with water (20ml). The mixture was basified with concentrated ammonia, the product extracted with ethyl acetate, dried over $MgSO_4$, filtered and the solvent evaporated. Chromatography on silica eluting with ethyl acetate/ethanol (20/1) gave the desired product, 227mg, mp. 251-253°C.
NMR analysis ($\delta$)
Solvent - DMSO-$d_6$
8.00(d,1H), 7.5(d,1H), 6.15-5.95(br.d,4H,-2$NH_2$) 1.8(s,3H).

Example 20

2-Amino-5-(2,3-dichlorophenyl)-4-(4-methyl-1-piperazinyl) pyrimidine monomesylate

2-Amino-4-chloro-5-(2,3-dichlorophenyl)pyrimidine (Example 13(vi), 0.5g) was stirred with N-methylpiperazine (2.5ml) in absolute ethanol (7ml) in a sealed vessel at 80°C for 15 hours. The resulting solution was evaporated to dryness and the residue chromatographed on silica eluting with chloroform and 5% methanol in chloroform. The crude product was suspended in sodium bicarbonate solution, stirred filtered, washed with water and dried at 80°C to give the title compound 0.475g.

0.232g of this material was suspended in absolute ethanol (10ml) and cooled in ice bath. Methanesulphonic acid (0.065g) was then added dropwise and stirring continued for 30 minutes. The resulting solution was evaporated to dryness at reduced pressure. The residue was dissolved in water, filtered and freeze-dried to give the salt 0.12g, mp. 120°C (indistinct).
NMR Analysis:
$\delta$H ($CDCl_3$); 2.25(7H,m), 3.30(4H,m), 4.85(2H,br.s.), 7.25(2H,m), 7.45(1H,dd), 7.75(1H,s).

Example 21

2-Amino-5-(2,3-dichlorophenyl)-4-(N-morpholino) pyrimidine

2-Amino-4-chloro-5-(2,3-dichlorophenyl)pyrimidine (Example 13(vi), 0.5g) was stirred with morpholine (2.5ml) in absolute ethanol (7ml) in a sealed vessel at 80°C for 15 hours. On cooling, a white solid separated from the reaction mixture. This was filtered off, washed with ether, and dried at 60°C. The crude product was recrystallised from propan-2-ol, and dried at 80°C to give the title compound 0.27g, mp. 174-176°C.
NMR Analysis:
$\delta$H ($CDCl_3$) 3.25(4H,m), 3.55(4H,dd), 4.85(2H,br.s), 7.25(2H,m), 7.45(1H,dd), 7.77(1H,s).

Example 22

4-Amino-2-(4-benzyl-1-piperazinyl)-5-(2,3,5-trichlorophenyl)pyrimidine

(i) N-Benzylpiperazinoformamidine hydroiodide

Made in an analogous manner to N-methylpiperazinoformamidine (Example 10(i)) from N-benzylpiperazine (Aldrich).

(ii) 4-Amino-2-(4-benzyl-1-piperazinyl)-5-(2,3,5-trichlorophenyl)pyrimidine

To a stirred solution of NaOEt (from 0.08g sodium) in ethanol (10ml) was added N-benzylpiperazinoformamidine hydroiodide (1.0g). After stirring for 10 minutes at room temperature 2-(2,3,5-trichlorophenyl)-3-methoxyacrylonitrile (Example 3(v), 0.38g) was added and the mixture stirred at reflux for 4½ hours. After standing overnight at room temperature the mixture was concentrated in vacuo, dissolved in dichloromethane and filtered. The filtrate was reconcentrated and the residue purified by chromatography on silica, eluting with $CHCl_3$:MeOH 10:1 to give the title compound as the free base, 0.47g.

The free base was dissolved in ethanol (10ml) and cooled in an ice bath. Methanesulphonic acid (70µl) was added and the solution stirred at room temperature for 1 hour. The solvent was evaporated and the residue triturated with ether, the solid was filtered, dissolved in cold water and freeze dried to give the title compound as its salt as a pale yellow solid, 0.26g, mp. 176-181°C dec.

NMR Analysis:

δH ($CDCl_3$) 2.50(4H,t), 3.55(2H,s), 3.82(4H,t), 4.5(2H,br.s.), 7.25-7.4(5H,m), 7.20(1H,d), 7.50(1H,d), 7.82-(1H,s).

Example 23 (Compound 25)

2,4-Diamino-5-(3,5-dichloro-4-aminophenyl)pyrimidine

(i) Methyl 4-amino-3,5-dichlorobenzoate

To a mixture of 4-Amino-3,5-dichlorobenzoic acid (Aldrich, 25g) and methanol (250ml) cooled to 5°C was added concentrated sulphuric acid (3.5ml) dropwise over 10 minutes. The mixture was heated at reflux for 20 hours and then left at room temperature over the weekend. The reaction mixture was concentrated in vacuo to a small volume, the solid was filtered, washed with methanol and petroleum ether and finally crystallised from aqueous methanol, 24.65g, mp. 76-78°C.

(ii) 4-Amino-3,5-dichlorobenzyl alcohol

The above ester (11.0g) was dissolved in dry tetrahydrofuran (150ml), lithium borohydride (1.1g) was added in portions and the mixture stirred at reflux for 23 hours. After cooling, the reaction mixture was concentrated in vacuo, partitioned between ethyl acetate (400ml) and water (50ml). The organic phase was washed with 1N sodium hydroxide solution (50ml) and brine (50ml), dried over $MgSO_4$, evaporated to dryness and finally crystallised from chloroform, 5.92g, mp. 107-109°C.

(iii) 3,5-Dichloro-4-(N,N-dimethylaminomethyleneamino)phenylacetonitrile

A mixture of 4-amino-3,5-dichlorobenzyl alcohol (17.8g), thionyl chloride (250ml) and DMF (7.8ml) was stirred at reflux for 4 hours. After cooling, the mixture was concentrated in vacuo slurried with ether and filtered. The solid was partitioned between ethyl acetate (400ml) and water (150ml) and the organic phase washed with 1M $Na_2CO_3$ solution and brine, dried over $MgSO_4$ and concentrated in vacuo to give 3,5-dichloro-4-(N,N-dimethylaminomethyleneamino)benzylchloride (19.92g) which was used without further purification.

To a vigorously stirred solution of 3,5-dichloro-4-(N,N-dimethylaminomethyleneamino)benzyl chloride (19.92g) in dichloromethane (85ml) was added a mixture of KCN (14.62g) and tetrabutylammonium hydrogen sulphate (0.5g) in water (85ml). After stirring at room temperature for 2½ days the mixture was diluted with dichloromethane, the organic phase washed with water (3 x 50ml) dried over $MgSO_4$ and concentrated in vacuo to leave an oil. This was chromatographed on $SiO_2$ using hexane/ether 7:3 as eluent.

The purified 3,5-dichloro-4-(N,N-dimethylaminomethyleneamino)phenylacetronitrile was obtained as colourless prisms on crystallisation from hexane/ether 2:1, 13.88g, mp. 71-73 °C.

(iv) 2-[3,5-Dichloro-4-(N,N-dimethylaminomethyleneamino)phenyl]-3-oxopropionitrile sodium salt

Prepared in an analogous manner to 2-(2,3,5-trichlorophenyl)-3-oxopropionitrile sodium salt (Example 3-(iv)), 0.87g and used without further purification.

(v) 2-[3,5-Dichloro-4-(N,N-dimethylaminomethyleneamino)phenyl]-3-methoxyacrylonitrile

Prepared in an analogous manner to 2-(2,3,5-trichlorophenyl)-3-methoxyacrylonitrile (Example 3(v)), 0.53g, mp. 121-126 °C.

(vi) 2,4-Diamino-5-(3,5-Dichloro-4-(N,N-dimethylaminomethyleneamino)phenyl]-pyrimidine

Prepared in an analogous manner to 2,4-Diamino-5-(2,3,5-trichlorophenyl)-6-methylpyrimidine (Example 3(vi)), 0.085g, mp. 202-203 °C.

(vii) 2,4-Diamino-5-(3,5-Dichloro-4-aminophenyl)pyrimidine

2,4-Diamino-5-(3,5-dichloro-4-(N,N-dimethylaminomethyleneamino)phenyl]-pyrimidine (55mg), ethanol (5ml), 0.880 ammonia (1.5ml) were sealed in a tube and stirred at 100 °C for 48 hours. After cooling the solvent was evaporated and the residue crystallised from MeCN, 48mg, mp. 199-201 °C.
NMR Analysis:
δH ($CDCl_3$); 4.50(2H,br.s.), 4.83(2H,br.s), 4.96(2H,br.s),
7.06(2H,s), 7.60(1H,s).

Example 24 (Compound 26)

2,4-Diamino-5-(3,5-dichloro-4-aminophenyl)-6-methylpyrimidine

(i) 2-[3,5-Dichloro-4-(N,N-dimethylaminomethyleneamino)phenyl]-3-oxobutyronitrile

To a stirred solution of NaOEt (from 0.4g sodium) in ethanol (10ml) was added 3,5-dichloro-4-(N,N-dimethylaminomethyleneamino)phenylacetonitrile (3.0g) and ethyl acetate (2.58g). The mixture was stirred at reflux for 5 hours then left standing overnight at room temperature. The mixture was then concentrated, the residue was dissolved in water (50ml) and washed with ether. The aqueous phase was acidified with concentrated $H_2SO_4$ and the resulting solid filtered off and washed with water. The damp solid was dissolved in ethyl acetate and basified with dilute ammonium hydroxide solution. The organic phase was dried over $MgSO_4$, concentrated in vacuo and used without further purification, 1.40g.

(ii) 2-[3,5-Dichloro-4-(N,N-dimethylaminomethyleneamino)phenyl]-3-methoxybut-2-enonitrile

Prepared in an analogous manner to 2-(2,3,5-trichlorophenyl)-3-methoxybut-2-enonitril (Example 10(iii)), 1.44g.

(iii) 2,4-Diamino-5-[3,5-dichloro-4-(N,N-dimethylaminomethyleneamino)phenyl]-6-methylpyrimidine

Prepared in an analogous manner to 2,4-diamino-5-(2,3,5-trichlorophenyl)-6-methylpyrimidine (Example 10(iv)) from guanidine hydrochloride 0.41 g mp. 283-286 °C.

(iv) 2,4-Diamino-5-(4-amino-3,5-dichlorophenyl)-6-methylpyrimidine

Prepared in an analogous manner to 2,4-diamino-5-(3,5-dichloro-4-aminophenyl)pyrimidine (Example 23 (vii)) 285mg, mp. 209-211 °C.
NMR Analysis:
δH (DMSO); 1.86(3H,s), 5.5(2H,br.s) 5.86(2H,br.s.), 6.2(2H,br.s), 7.03(2H,s).

Example 25 (Compound 27)

2,4-Diamino-5-(3,5-dichloro-4-aminophenyl]-6-trifluoromethylpyrimidine

(i) 2-[3,5-Dichloro-4-(N,N-dimethylaminomethyleneamino)phenyl]-4,4,4,trifluoro-3-oxobutyronitrile

To a stirred solution of NaOEt (from 0.69g sodium) in ethanol (20ml) was added 3,5-dichloro-4-(N,N-dimethylaminomethyleneamino)phenylacetonitrile (6.4g) and ethyltrifluoroacetate (4.43g). The mixture was stirred at reflux for 5 hours, cooled and then concentrated, the residue was dissolved in water (80ml) and washed with ether. The aqueous phase was acidified with concentrated $H_2SO_4$ and the resulting solid filtered off and washed with water and dried (9.08g). The solid was suspended in water basified with dilute ammonium hydroxide solution and concentrated in vacuo. The residue was dissolved in acetone, dried over $MgSO_4$ and evaporated and finally slurried with ether, filtered washed and dried 7.8g, mp. 195-200 °C.

(ii) 2-[3,5-Dichloro-4-(N,N-dimethylaminomethyleneamino)phenyl]-4,4,4,trifluoro-3-methoxybut-2-enonitrile

Prepared in an analogous manner to 2-(2,3,5-trichlorophenyl)-3-methoxybut-2-enonitrile (Example 10(iii)) 1.95g.

(iii) 2,4-Diamino-5-[3,5-dichloro-4-(N,N-dimethylaminomethyleneamino)phenyl]-6-trifluoromethylpyrimidine

Prepared in an analogous manner to 4-amino-2-(4-methylpiperazin-1-yl-5-(2,3,5-trichlorophenyl)-6-trifluoromethylpyrimidine (Example 10(iv)) from guanidine hydrochloride 0.5g, mp. 239-241 °C.

(iv) 2,4-Diamino-5-(3,5-dichloro-4-aminophenyl)-6-trifluoromethylpyrimidine

Prepared in an analogous manner to 2,4-diamino-5-(3,5-dichloro-4-aminophenyl)pyrimidine (Example 23 (vii)) 350mg, mp. 216-219 °C.
NMR Analysis:
δH (DMSO); 5.55(2H,s), 6.25(2H,br.s), 6.42(2H,br.s), 7.00(2H,s).

Example 26 (Compound 28)

4-Amino-2-(4-n-butyl-1-piperazinyl)-5-(2,3,5-trichlorophenyl)pyrimidine

(i) N-Butylpiperazine

N-Benzylpiperazine (Aldrich, 8.8g) was dissolved in ethanol (150ml) followed by addition of sodium bicarbonate (21g) and butyl iodide (BDH, 8.6ml). The mixture was heated at reflux for 3 hours then left overnight at room temperature. The insoluble material was removed by filtration and the filtrate concentrated in vacuo. The residue was dissolved in ethyl acetate (400ml), washed with water (3 x 70ml), dried over $MgSO_4$ and concentrated in vacuo. The residue was dissolved in ether, filtered through Hyflo and reconcentrated to give the desired intermediate as a yellow liquid, 10.43g.

A solution of the above piperazine in MeOH (100ml) was reduced under an atmosphere of hydrogen in the presence of 10% Pd\C (0.5g). The mixture was filtered and the filtrate concentrated to give the intermediate as a colourless oil, 2.32g, bpt. 40-44 ° 0.2mm Hg.

(ii) N-Butylpiperazinoformamidine sulphate

Butylpiperazine (0.71g) was dissolved in water (10ml), 2-methyl-2-thiopseudourea sulphate (Eastman, 0.52g) was added and the solution was stirred whilst $N_2$ was bubbled through, at room temperature for 21 hours. The solution was concentrated in vacuo. The residue was triturated with i-PrOH filtered and dried in vacuo, 0.575g, mp. 243-249 °C.

(iii) 4-Amino-2-(4-n-butyl-1-piperazinyl)-5-(2,3,5-trichlorophenyl)pyrimidine

Prepared in an analogous manner to 4-amino-2-(4-benzyl-1-piperazin-1-yl)-5-(2,3,5-trichlorophenyl)-pyrimidine (Example 22) by reacting the above formamidine with 2-(2,3,5-trichlorophenyl)-3-methoxyacrylonitrile (Example 3(v)) and subsequent conversion to the dimesylate salt by dissolving in ether and adding 2 equivalents methanesulphonic acid, mp. 205° with decomposition.
NMR Analysis:
δH (DMSO); 0.95(3H,t), 1.38(2H,m), 1.72(2H,m) 2.36(6H,s) 2.36(6H,s) 3.1-3.8(9H,m), 4.5-4.65(2H,br.d), 7.5-(1H,d), 7.92(1H,s), 7.95(1H,d).

Example 27 (Compound 29)

4-Amino-2-(4-ethyl-1-piperazinyl)-5-(2,3,5-trichlorophenyl)pyrimidine

(i) N-ethylpiperazinoformamidine hydroiodide

The title compound was prepared in an analogous manner to N-methylpiperazinoformamidine (Example 10(i)) from ethylpiperazine (BASF) except that the residue was triturated with ethyl acetate, 6.9g, mp. 151-153°C.

(ii) N-ethylpiperazinoformamidine acetate

1-Formamidino-2,5-dimethylpyrazole acetate (ex Aldrich, 1.89g), 1-ethylpiperazine (BASF, 1.33g) and triethylamine (1.61ml) are dissolved in dichloromethane (25ml) and stirred at room temperature for 18 hours. The white suspension was filtered washed with dichloromethane and dried in vacuo 1.35g, mp. 240-241°C.

(iii) 4-Amino-2-(4-ethyl-1-piperazinyl)-5-(2,3,5-trichlorophenyl)pyrimidine

This compound was prepared in an analogous manner to 4-amino-2-(4-benzyl-1-piperazinyl)-5-(2,3,5-trichlorophenyl)pyrimidine (Example 22) by reacting the above formamidine hydroiodide with 2-(2,3,5-trichlorophenyl)-3-methoxyacrylonitrile (Example 3(v)) and subsequent conversion to the dimesylate salt by dissolving in ether and adding 2 equivalents methanesulphonic acid, mp. 213-215°C.
NMR Analysis:
δH ($CDCl_3$) 1.14 (3H,t), 2.4-2.6(6H,m), 3.84(4H,t), 4.55(2H,br.s.), 7.22(1H,d), 7.50(1H,d), 7.82(1H,s).

Example 28 (Compound 30)

4-Amino-2-(4-isopropyl-1-piperazinyl)-5-(2,3,5-trichlorophenyl)pyrimidine

(i) N-isopropylpiperazinoformamidine hydroiodide

Isopropylpiperazine dihydrochloride (Emka-chemie, 5.03g) was dissolved in water (15ml) and passed onto a column of IR410 OH form and eluted with water. The eluent was concentrated in vacuo dissolved in ether, dried over $MgSO_4$ and evaporated to give the base as a pale yellow oil (1.86g) which was used without further purification.

The title compound was prepared in an analogous manner to N-methylpiperazinoformamidine (Example 10(i)) using the above free base except that the residue was triturated with acetone, 0.1g.

(ii) 4-Amino-2-(4-isopropyl-1-piperazinyl)-5-(2,3,5-trichlorophenyl)pyrimidine

This compound was prepared in an analogous manner to 4-amino-2-(4-benzyl-1-piperazinyl)-5-(2,3,5-trichlorophenyl)pyrimidine (Example 22) by reacting the above formamidine hydroiodide with 2-(2,3,5-trichlorophenyl)-3-methoxyacrylonitrile (Example 3(v)) and subsequent conversion to the dimesylate salt by dissolving in ether and adding 2 equivalents methanesulphonic acid, mp. 130°C with decomposition.
NMR Analysis:
δH (DMSO); 1.60(6H,d), 2.4-2.55(4H,m), 2.7(1H,m), 3.6-3.75(4H,m), 6.3(2H,br.s.), 7.36(1H,d.), 7.65(1H,s), 7.80(1H,d).

Example 29 (Compound 31)

4-Amino-2-(4-cyclopropyl-1-piperazinyl)-5-(2,3,5-trichlorophenyl)pyrimidine

(i) N-cyclopropylpiperazinofirmamidine hydroiodide

Cyclopropylpiperazine dihydrochloride (Emka-chemie, 10.0g) was dissolved in water (20ml) and passed onto a column of IR410 OH form and eluted with water. The eluent was concentrated in vacuo dissolved in acetone, dried over $MgSO_4$ and evaporated to give the base as a pale green oil (4.41g) which was used without further purification.

The title compound was prepared in an analogous manner to N-methylpiperazinoformamidine (Example 10(i)) using the above free base except that the residue was triturated with ether, 1.84g, mp. 127-133°C dec.

(ii) 4-Amino-2-(4-cyclopropyl-1-piperazinyl)-5-(2,3,5-trichlorophenyl)pyrimidine

This compound was prepared in an analogous manner to 4-Amino-2-(4-benzylpiperazin-1-yl)-5-(2,3,5-trichlorophenyl)pyrimidine (Example 22) by reacting the above formamidine hydroiodide with 2-(2,3,5-trichlorophenyl)-3-methoxyacrylonitrile (Example 3(v)) and subsequent conversion to the dimesylate salt by dissolving in ether and adding 2 equivalents methanesulphonic acid, mp. 214-217°C.
NMR Analysis:
δH (DMSO); 0.8-0.95(2H,m), 0.95-1.06(2H,m), 2.4(6H,s), 2.9(1H,m), 3.1-3.7(m), 7.45(1H,d), 7.9(1H,s), 7.95-(1H,d).

Example 30 (Compound 32)

4-Amino-2-(4-ethyl-1-piperazinyl)-5-(3,5-dichlorophenyl)-6-trifluoromethylpyrimidine dimesylate

2-(3,5-dichlorophenyl)-3-oxo-3-trifluoromethylpropionitrile (Example 1(ii)) (759mg) was dissolved in triethylorthoformate (3.5ml) and placed in an oil bath at 146°C. The temperature of the oil bath was raised until the reaction mixture was refluxing, and this temperature was maintained for 4½ hours. The volatile products were then distilled of in vacuo to leave the enol ether which was used without further purification (868mg).

To a stirred solution of NaOEt (from 152mg sodium) in ethanol (10ml) was added N-ethylpiperazinoformamidine hydroiodide (1.4gms) in portions over 10 minutes. After stirring for 10 minutes at room temperature a solution of the above enol ether in ethanol (5ml) was added and the mixture stirred at reflux for 6 hours. After standing overnight at room temperature the mixture was concentrated in vacuo, dissolved in dichloromethane, and filtered. The filtrate was reconcentrated and the residue purified by chromatography on silica eluting with dichloromethane and 5% MeOH to give the desired product (674mg).

The free base was dissolved in ethanol/ether and methanesulphonic acid (220μl) added. The resulting precipitate was collected, washed and dried in vacuo. The solid was then dissolved in water, filtered, and freeze-dried to give the title compound as a white solid (695mg). mpt 256-7°C.
NMR Analysis:
δH (DMSO); 1.28(3H,t), 2.40(6H,s), 2.9-3.4 (6H,m), 3.6(2H,br.s), 4.7(2H,br.s), 7.25(2H,d), 7.68(1H,t).

Pharmacological Activity

Inhibition of Glutamate release and Inhibition of Rat Liver DHFR

Compounds according to the invention were tested for their effect on veratrine-evoked release of glutamate from rat brain slices according to the protocol described in Epilepsia 27(5): 490-497, 1986. The protocol for testing for inhibition of DHFR activity was a modification of that set out in Biochemical Pharmacology Vol. 20 pp 561-574, 1971.

The results are given in Table 1, the $IC_{50}$ being the concentration of compound to cause 50% inhibition of (a) veratrine-evoked release of glutamate and (b) of DHFR enzyme activity.

TABLE 1

| Compound No. | $IC_{50}$($\mu$M) Glutamate Release (P95 limits) | $IC_{50}$($\mu$M) Rat Liver DHFR (P95 limits) |
|---|---|---|
| 1 | <10 | 4.59(1.9-10.75) |
| 2 | <10 | >100 |
| 3 | 2.5(1.2-4.9) | >100 |
| 4 | <10 | 100 |
| 5 | <10 | <100 |
| 6 | 10 | >100 |
| 7 | <10 | >100 |
| 8 | 0.27(0.04-1.65) | >100 |
| 9 | <10 | >100 |
| 10 | >10 | >100 |
| 11 | <10 | >100 |
| 12 | <10 | 13.8(6.9-27.3) |
| 14 | 0.137(0.026-0.79) | >100 |
| 15 | <10 | >100 |
| 16 | 10 | >100 |
| 17 | 2.17(1.5-2.9) | >100 |
| 18 | >10 | >100 |
| 19 | <10 | 5.558(1.58-19.77) |
| 20 | 10 | >100 |
| 21 | 10 | >100 |
| 22 | >10 | <100 |
| 25 | >10 | 0.034(0.013-0.08) |
| 26 | <10 | 0.034(0.015-0.07) |
| 27 | 10 | 1.54 |
| 28 | <10 | >100 |
| 29 | 2.56(1.34-4.87) | >100 |
| 30 | <10 | 100 |
| 31 | 10 | >100 |
| 32 | 2.43(0.84-7.01) | >100 |

Pharmaceutical Formulation Example

A: Tablets:

| | |
|---|---|
| Compound of Example 1 | 150 mg ) |
| Lactose | 200 mg ) |
| Maize Starch | 50 mg ) |
| Polyvinylpyrrolidone | 4 mg ) |
| Magnesium Stearate | 4 mg ) |
| ) = contents per tablet. | |

The drug was mixed with the lactose and starch and granulated with a solution of the polyvinylpyrrolidone in water. The resultant granules were dried, mixed with magnesium stearate and compressed to give tablets.

B: Injections

Injection I

The salt of the compound according to the invention was dissolved in sterile water for injection.

Intravenous injection formulation II

| | |
|---|---|
| Active ingredient | 0.20g |
| Sterile, pyrogen-free phosphate buffer (pH9.0) to | 10ml |

The compound of Example 1 as a salt is dissolved in most of the phosphate buffer at 35-40 °C, then made up to volume and filtered through a sterile micropore filter into sterile 10ml glass vials (Type 1) which are sealed with sterile closures and overseals.

In the following examples, the active compound may be any compound according to the invention or a pharmaceutically acceptable salt thereof.

C: Capsule formulations

Capsule Formulation I

Formulation I may be prepared by admixing the ingredients and filling two-part hard gelatin capsules with the resulting mixture.

| | mg/capsule |
|---|---|
| (a) Active ingredient | 250 |
| (b) Lactose B.P. | 143 |
| (c) Sodium Starch Glycollate | 25 |
| (d) Magnesium Stearate | 2 |
| | 420 |

Capsule Formulation II

| | mg/capsule |
|---|---|
| (a) Active Ingredient | 250 |
| (b) Macrogel 4000 BP | 350 |
| | 600 |

Capsules may be prepared by melting the Macrogel 4000 BP, dispersing the active ingredient in the melt, and filling two-part hard gelatin capsules therewith.

Capsule Formulation III (Controlled release capsule)

| | mg/capsule |
|---|---|
| (a) Active Ingredient | 250 |
| (b) Microcrystalline Cellulose | 125 |
| (c) Lactose BP | 125 |
| (d) Ethyl Cellulose | 13 |
| | 513 |

The controlled-release capsule formulation may be prepared by extruding mixed ingredients (a) to (c) using an extruder, then spheronising and drying the extrudate. The dried pellets are coated with ethyl cellulose (d) as a controlled-release membrane and filled into two-part hard gelatin capsules.

D: Syrup formulation

| | |
|---|---|
| Active ingredient | 0.2500 g |
| Sorbitol Solution | 1.5000 g |
| Glycerol | 1.0000 g |
| Sodium Benzoate | 0.0050 g |
| Flavour | 0.0125 ml |
| Purified Water q.s. to | 5.0 ml |

The sodium benzoate is dissolved in a portion of the purified water and the sorbitol solution added. The active ingredient is added and dissolved. The resulting solution is mixed with the glycerol and then made up to the required volume with the purified water.

E: Suppository formulation

| | mg/suppository |
|---|---|
| Active ingredient (63µl)* | 250 |
| Hard Fat, BP (Witepsol H15 - Dynamit Nobel) | 1770 |
| | 2020 |

* The active ingredient is used as a powder wherein at least 90% of the particles are of 63µm diameter or less.

One fifth of the Witepsol H15 is melted in a steam-jacketed pan at 45°C maximum. The active ingredient is sifted through a 200µm sieve and added to the molten base with mixing, using a Silverson fitted with a cutting head, until a smooth dispersion is achieved. Maintaining the mixture at 45°C, the remaining Witepsol H15 is added to the suspension which is stirred to ensure a homogenous mix. The entire suspension is then passed through a 250µm stainless steel screen and, with continuous stirring, allowed to cool to 40°C. At a temperature of 38-40°C, 2.02g aliquots of the mixture are filled into suitable plastic moulds and the suppositories allowed to cool to room temperature.

**Claims**

**1.** A pyrimidine selected from the following group and acid addition salts of such pyrimidines:-
2,4-diamino-5-(3,5-dichlorophenyl)-6-trifluoromethyl-pyrimidine;
2,4-diamino-6-methyl-5-(2,3,6-trichlorophenyl)pyrimidine;
2,4-diamino-5-(4-amino-2,3,5-trichlorophenyl)pyrimidine;
4-amino-2-(4-methyl-1-piperazinyl)-6-methyl-5-(3,4,5-trichlorophenyl)pyrimidine;
2,4-diamino-5-(3,4,5-trichlorophenyl)-6-methyl-pyrimidine;

4-amino-2-diethylamino-5-(2,3,5-trichlorophenyl)pyrimidine;
4-amino-2-(2-iso-propylamino)-5-(2,3,5-trichlorophenyl)pyrimidine;
4-amino-5-(3,5-dichlorophenyl)-2-(4-methyl-1-piperazinyl)pyrimidine;
2,4-diamino-5-(4-iodo-2,3,5-trichlorophenyl)pyrimidine;
4-amino-2-(4-methyl-4-oxide-1-piperazinyl)-5-(2,3,5-trichlorophenyl)-6-trifluoromethyl-pyrimidine;
4-amino-5-(3,5-dichlorophenyl)-6-methoxymethyl-2-(4-methyl-1-piperazinyl)pyrimidine;
2,4-diamino-5-[4-(N',N'-dimethylsulphamoyl)-2,3,5-trichlorophenyl]pyrimidine;
2-amino-5-(2,3-dichlorophenyl)-4-(1-piperidinyl)-pyrimidine;
4-amino-5-(3,5-dichlorophenyl)-2-(4-methyl-1-piperazinyl)-6-trifluoromethyl-pyrimidine;
2,4-diamino-5-(2,3,4,5-tetrachlorophenyl)pyrimidine;
cis 4-amino-2-(3,5-dimethyl-1-piperazinyl)-5-(2,3,5-trichlorophenyl)pyrimidine;
4-amino-2-(4-methyl-4-oxide-1-piperazinyl)-5-(2,3,5-trichlorophenyl)pyrimidine;
2,4-diamino-5-(3,5-dichloro-2-nitrophenyl)-6-methyl-pyrimidine;
2-amino-5-(2,3-dichlorophenyl)-4-(4-methyl-1-piperazinyl)pyrimidine;
2-amino-5-(2,3-dichlorophenyl)-4-(N-morpholino)pyrimidine;
4-amino-2-(4-benzyl-1-piperazinyl)-5-(2,3,5-trichlorophenyl)pyrimidine;
4-amino-2-(N-morpholino)-5-(3,5-dichlorophenyl)-6-methyl-pyrimidine;
4-amino-2-(N-morpholino)-5-(3,5-dichlorophenyl)-6-trifluoromethyl-pyrimidine;
2,4-diamino-5-(3,5-dichloro-4-aminophenyl)pyrimidine;
2,4-diamino-5-(3,5-dichloro-4-aminophenyl)-6-methyl pyrimidine;
2,4-diamino-5-(3,5-dichloro-4-aminophenyl)-6-trifluoromethyl-pyrimidine;
4-amino-2-(4-n-butyl-1-piperazinyl)-5-(2,3,5-trichlorophenyl)pyrimidine;
4-amino-2-(4-ethyl-1-piperazinyl)-5-(2,3,5-trichlorophenyl)pyrimidine;
4-amino-2-(4-isopropyl-1-piperazinyl)-5-(2,3,5-trichlorophenyl)pyrimidine;
4-amino-2-(4-cyclopropyl-1-piperazinyl)-5-(2,3,5-trichlorophenyl)pyrimidine;
4-amino-2-(4-ethyl-1-piperazinyl)-5-(3,5-dichlorophenyl)-6-trifluoromethyl-pyrimidine;
4-amino-2-(4-isopropyl-1-piperazinyl)-5-(3,5-dichlorophenyl)-6-trifluoromethyl-pyrimidine;
4-amino-2-(4-cyclopropyl-1-piperazinyl)-5-(3,5-dichlorophenyl)-6-trifluoromethyl-pyrimidine; and
4-amino-2-(4-methyl-1-piperazinyl)-5-(2,4,5-trichlorophenyl)pyrimidine.

**2.** A pyrimidine as claimed in claim 1 which is 2,4-diamino-5-(4-amino-2,3,5-trichlorophenyl)pyrimidine;
4-amino-5-(3,5-dichlorophenyl)-2-(4-methyl-1-piperazinyl)pyrimidine; or
4-amino-2-(4-ethyl-1-piperazinyl)-5-(2,3,5-trichlorophenyl)pyrimidine;
or an acid addition salt thereof.

**3.** A process for the production of a pyrimidine as claimed in claim 1 or an acid addition salt thereof, which process comprises:
(A) reacting an appropriate compound of formula (I)

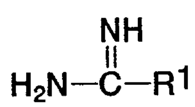

(I)

or a salt thereof, with an appropriate compound of formula (II)

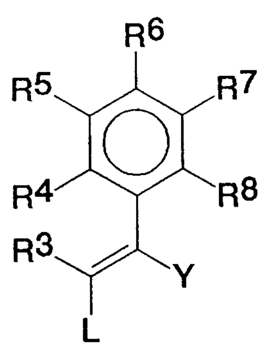

(II)

wherein $R^1$ and $R^3$ to $R^8$ take appropriate meanings for the pyrimidine to be produced; Y is cyano, carboxy, carbonyl or alkoxycarbonyl; and L is a leaving group; either or both of the compounds of formulae (I) and (II) optionally being used in the form of a protected derivative thereof, followed if necessary by removal of any protecting groups present; or

(B) reacting a compound of formula (I), or a salt thereof, with a compound of formula (III)

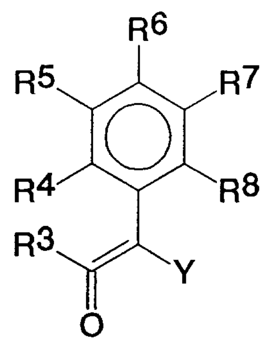

(III)

wherein $R^1$ and $R^3$ to $R^8$ take appropriate meanings for the pyrimidine to be produced; Y is cyano, carboxy, carbonyl or alkoxycarbonyl; either or both of the compounds of formulae (I) and (III) optionally being used in the form of a protected derivative thereof, followed if necessary by removal of any protecting groups present; or

(C) reacting a compound of formulae (I), or a salt thereof, with a compound of formula (IV)

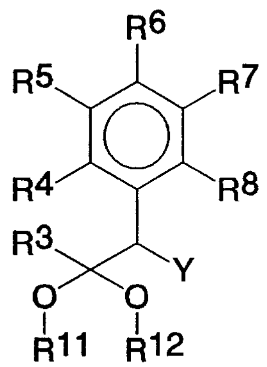

(IV)

wherein $R^1$ and $R^3$ to $R^8$ take appropriate meanings for the pyrimidine to be produced; Y is cyano, carboxy, carbonyl or alkoxycarbonyl; $R^{11}$ and $R^{12}$ are each alkyl or taken together form a group $-(C(R)_2)_n-$ where n is 2 to 4 and each R is independently hydrogen or alkyl; either or both of the compounds of formulae (I) and (IV) optionally being used in the form of a protected derivative thereof, followed if necessary by removal of any protecting groups present; or

(D) dehydrogenating the corresponding dihydropyrimidine or a protected derivative thereof, followed if necessary by removal of any protecting groups present; or

(E) interconversion of one pyrimidine as claimed in claim 1 into another such pyrimidine;

optionally followed by conversion of the pyrimidine produced in the form of the free base into an acid addition salt or conversion of one acid addition salt to another.

4. A pharmaceutical composition which comprises a pyrimidine as claimed in claim 1 or a pharmaceutically acceptable acid addition salt thereof together with at least one pharmaceutically acceptable carrier.

European Patent Office

# EUROPEAN SEARCH REPORT

Application Number
EP 95 11 1314

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,P, X | EP-A-0 372 934 (WELLCOME)<br>* page 1 - page 11; claims; examples 1,3 *<br>----- | 1,2,8-11 | C07D239/48<br>A61K31/505 |
| | | | TECHNICAL FIELDS SEARCHED (Int.Cl.5) |
| | | | C07D |

The present search report has been drawn up for all claims

| Place of search | Date of completion of the search | Examiner |
|---|---|---|
| THE HAGUE | 30 August 1995 | Francois, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document